(19) Europäisches Patentamt
European Patent Office
Office européen des brevets

(11) Veröffentlichungsnummer: **0 464 259 A2**

(12) # EUROPÄISCHE PATENTANMELDUNG

(21) Anmeldenummer: **90124136.4**

(51) Int. Cl.$^5$: **C07C 33/025**, C07C 29/124

(22) Anmeldetag: **13.12.90**

(30) Priorität: **06.07.90 DE 4021578**

(43) Veröffentlichungstag der Anmeldung:
**08.01.92 Patentblatt 92/02**

(84) Benannte Vertragsstaaten:
**BE CH DE FR GB LI NL**

(71) Anmelder: **HÜLS AKTIENGESELLSCHAFT**
**- RSP Patente / PB 15 - Postfach 13 20**
**W-4370 Marl 1(DE)**

(72) Erfinder: **Wachholz, Gerhard, Dr.**
**Lipper Weg 193**
**W-4370 Marl(DE)**
Erfinder: **Voges, Heinz-Werner, Dr.**
**Im Gorden 45**
**W-4270 Dorsten(DE)**

(54) **Verfahren zur Herstellung von Dihydromyrcenol aus Dihydromyrcenylchlorid.**

(57) Die Erfindung betrifft ein Verfahren zur Herstellung von Dihydromyrcenol aus Dihydromyrcenylchlorid, wobei man bei der basischen Hydrolyse einen Phasentransferkatalysator in einer Menge von 0.001-10 Mol-%, bezogen auf Dihydromyrcenylchlorid, zusetzt.

Das so erzeugte Dihydromyrcenol wird als Riechstoff verwendet.

EP 0 464 259 A2

Die Erfindung betrifft eine verbesserte und wesentlich vereinfachte Methode zur Darstellung von Dihydromyrcenol, wobei das Dihydromyrcenylchlorid als Edukt eingesetzt wird.

Bei Dehydromyrcenol handelt es sich um ein Monoterpen, das in der Riechstoffindustrie eine vielfältige Anwendung findet. Von besonderem Interesse sind ferner seine Folgeprodukte wie Ether und Ester.

Dihydromyrcenylchlorid wird durch Hydrolyse in Gegenwart eines basischen Puffers direkt in den Alkohol überführt. Bei dieser Hydratisierungsreaktion handelt es sich um einen wichtigen Teilschritt in der Synthesesequenz, die von dem Naturstoff α-Pinen über cis-Pinan, Dihydromyrcen und Dihydromyrcenylchlorid zu dem begehrten Riechstoff Dihydromyrcenol führt.

Es ist bekannt und in einer Vielzahl von Arbeiten beschrieben, daß sich Dihydromyrcenol ausgehend von α-Pinen über diese mehrstufige Synthese darstellen läßt:

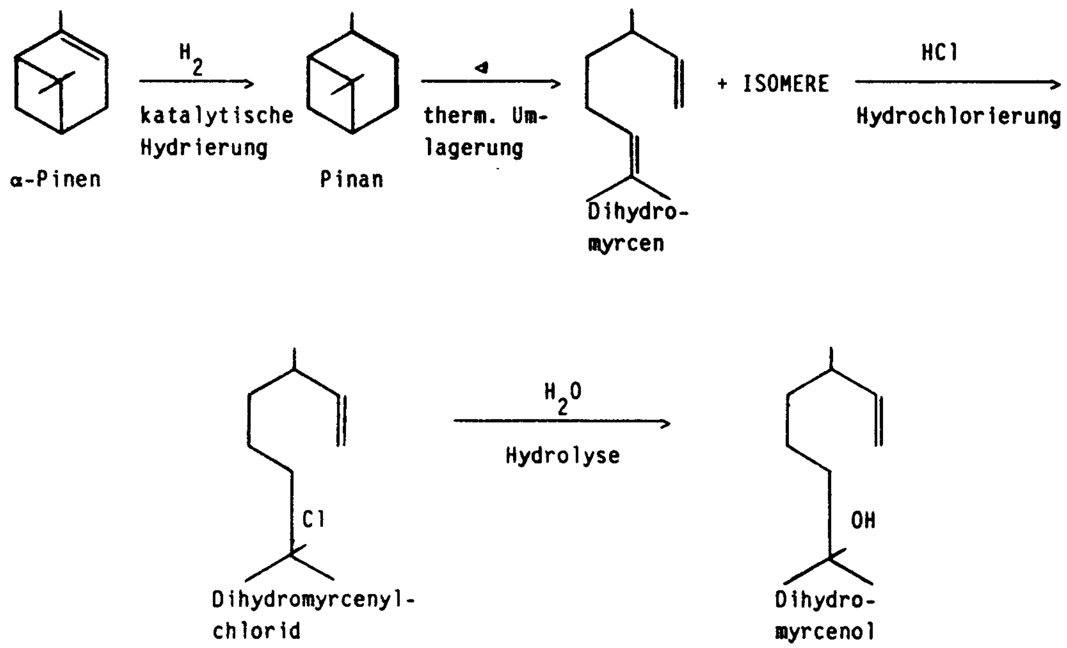


α-Pinen

Pinan

Dihydromyrcen

Dihydromyrcenylchlorid

Dihydromyrcenol

α-Pinen wird zu Pinan hydriert, wobei das cis-Isomere das für die nachfolgende thermische Umlagerung weitaus wichtigere ist, läßt es sich doch selektiver in das Dihydromyrcen überführen. Beschrieben wird die Hydrierung von W. Cocker, J. Chem. Soc. (C), 41-47 (1966) sowie in US-PS 4 018 842 und US-PS 4 310 714.

Cis-Pinan läßt sich in der Gasphase bei Temperaturen von 420-600 °C thermisch umlagern, wobei das Dihydromyrcen als Hauptprodukt zugänglich wird. Daneben entstehen aber auch eine ganze Reihe von Strukturisomeren:

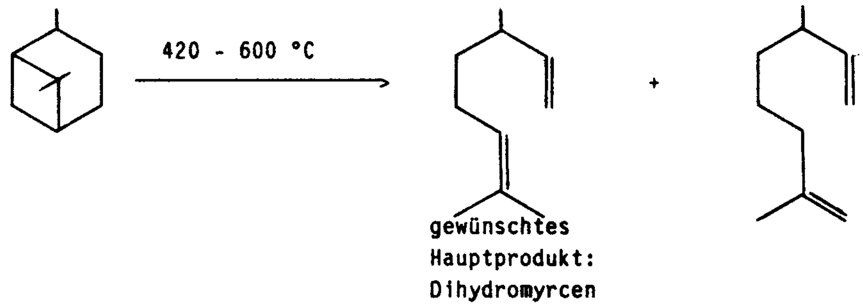


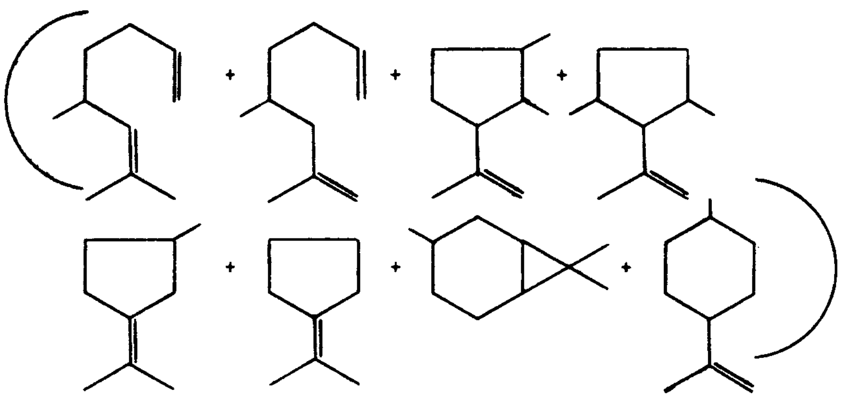

Beschrieben wird diese thermische Umlagerung in einer Reihe von Patenten, so u. a. in US-PS 2 388 084, US-PS 2 902 495,
US-PS 3 277 206, DE-PS 1 149 000, und in DE-PS 2 744 386. In der wissenschaftlichen Literatur fand diese Reaktion schon früh ein sehr großes Interesse, folgende Arbeiten belegen dies; V. N. Jpatieff, JACS 75, 6222-6225 (1953), W. D. Huntsman, JACS 80, 2252-2254 (1957), H. Pines, JACS 76, 4412-4416 (1954) und J. Tanaka, Bull. Chem. Soc., Japan 44, 130-132 (1971).

Die Darstellung des Dihydromyrcenols selbst erfolgt sehr selektiv und mit sehr guten Ausbeuten über Dihydromyrcenylchlorid und anschließende Hydrolyse. Die Hydrochlorierung des Dihydromyrcens kann durch Einleiten von trockenem HCl-Gas in Dihydromyrcen erfolgen, wie in der GB-PS 859 567 beschrieben. Des weiteren sind zur Verbesserung der Reaktion verschiedene Katalysatoren vorgeschlagen worden, so z. B. Kupferchlorid (JA 60 058 930) sowie Lewis- und Protonensäuren (EP-A-0 170 205). Allen Hydrochlorierungsverfahren gemein ist, daß man sowohl von reinem Dihydromyrcen wie auch von dem rohen Produktgemisch der thermischen Umlagerung ausgehen kann.

Die Hydrolyse des erhaltenen Chlorids erfolgte bisher meist durch Zusatz von NaOH, $Ca(OH)_2$ oder $CaCO_3$ und $Na_2CO_3$, so beschrieben in den Patenten GB-PS 859 567, US-PS 2 882 323. Neuerdings wird der Zusatz von sogenannten Hydroxylierungskatalysatoren, wie z. B. ZnO, näher beschrieben (EP-A-0 170 205). Aus den Beispielen der Patentschrift EP-A-0 170 205 geht hervor, daß der "Hydroxylierungskatalysator" ZnO in einer Menge von 0.6 Mol pro 1 Mol Dihydromyrcenylchlorid eingesetzt wird.

Bei den Reaktionsschritten von α-Pinen zum Dihydromyrcenylchlorid handelt es sich um ausgearbeitete und laufend verbesserte Verfahren, die heute zum Stand der Technik gerechnet werden. Um so erstaunlicher ist es, daß für die Hydrolyse bisher keine ebenso gut ausgearbeiteten Verfahren vorliegen, obwohl es sich hier um die zum gewünschten Zielprodukt führende letzte und entscheidende Stufe einer doch recht aufwendigen Synthesesequenz handelt.

Von großem Nachteil sind hier vor allem die nach der Lehre des Standes der Technik erforderlichen sehr langen Hydrolysezeiten. So befinden sich im Reaktionsgemisch, wie Z. B. in GB-PS 859 567 beschrieben, nach 15 Stunden unter Rückflußbedingungen noch 5-10 % Dihydromyrcenylchlorid. Um einen weiter verringerten Restgehalt an Dihydromyrcenylchlorid zu erreichen, würden die Hydrolysezeiten noch beträchtlich verlängert werden müssen. Umsetzungen bis zu einem Dihydromyrcenylchloridrestgehalt von < 0.1 % sind nach diesem Verfahren nur nach extrem langen Reaktionszeiten von ca. 3-6 Tagen möglich.

Ein weiterer negativer Aspekt ist die sehr aufwendige Trennung des gewünschten Dihydromyrcenols von nicht umgesetztem Chlorid. In einem neueren Verfahren (EP-A-0 170 205) wird beschrieben, daß eine

Verbesserung durch sogenannte Hydroxylierungsreagenzien zu erzielen sei, wobei hier beispielsweise Zink- und Magnesiumoxid zu nennen wären. Eine Verbesserung wird allerdings nur teilweise erreicht, die Hydrolysezeiten sind weiterhin sehr lang. In einem typischen Beispiel wird nach 11 Stunden unter Rückflußbedingungen nur ein Umsatz des Dihydromyrcenylchlorids von ca. 70 % erreicht.

Der geringe, wenn überhaupt feststellbare Fortschritt der Lehre der EP-A-0 170 205 gegenüber der GB-PS 859 567 wird dadurch zunichte, daß Phasentrennprobleme bei der Aufarbeitung des Produktgemisches nach erfolgter Hydrolyse auftreten, offenbar dadurch verursacht, daß pro 0.5 Mol Dihydromyrcenylchlorid nicht weniger als 0.3 Mol Zinkoxid eingesetzt werden, das sich offenbar weitgehend in Zinkchlorid umwandelt.

Der vorliegenden Erfindung liegt die Aufgabe zugrunde, die wichtigste letzte Stufe in der Synthesesequenz vom α-Pinen zum Dihydromyrcenol wesentlich zu verbessern. Unter dieser Zielsetzung war ein Verfahren zu finden, das einen möglichst vollständigen Umsatz des Dihydromyrcenylchlorids in wesentlich kürzeren Reaktionszeiten ermöglichte. Weiterhin sollte durch einen möglichst quantitativen Umsatz des Chlorids erreicht werden, eine wesentlich vereinfachte destillative Aufarbeitung des Reaktionsproduktes möglich zu machen. Eine weitere Forderung bestand darin, daß nach erfolgter Hydrolyse des Chlorids Phasentrennprobleme im Zuge der Produktaufarbeitung vermieden werden sollten.

Die Aufgabe wird erfindungsgemäß dadurch gelöst, daß eine Beschleunigung der Hydrolyse durch Zusatz von Phasentransferkatalysatoren zum Reaktionsgemisch, das aus Dihydromyrcenylchlorid und einer Base enthaltenden Wasserphase besteht, erreicht wird. Hierbei handelt es sich um quarternäre Salze, bevorzugt um quarternäre Ammonium-, Phosphonium- und Pyridiniumsalze.

Gegenstand der Erfindung ist ein Verfahren zur Herstellung von Dihydromyrcenol aus Dihydromyrcenylchlorid, welches dadurch gekennzeichnet ist, daß bei der basischen Hydrolyse ein Phasentransferkatalysator in einer Menge von 0.001-10 Mol-%, bezogen auf Dihydromyrcenylchlorid, zugesetzt wird.

Eine Reihe von Phasentransferkatalysatoren wurden auf ihre Anwendbarkeit hin überprüft. Dabei zeigte sich, daß noch relativ geringfügige Zusätze solcher Stoffe die Hydrolysegeschwindigkeit in überraschendem Maße erhöhen, wie im folgenden näher ausgeführt wird:

Führt man die Hydrolyse in herkömmlicher Weise durch, d. h. indem man Dihydromyrcenylchlorid mit einer wäßrigen Lösung von Natriumhydrogencarbonat oder anderen basisch wirkenden Verbindungen zur Reaktion bringt, so wird, wie im Vergleichsbeispiel näher beschrieben, eine Reaktionszeit von 22 Stunden unter Rückflußbindungen (ca. 100 °C) benötigt. Selbst nach dieser Zeit verbleibt ein Dihydromyrcenylchloridrestgehalt von 4 % im Reaktionsgemisch. Durch Zusatz von nur 0.5 Mol-% (bezogen auf Dihydromyrcenylchlorid) Hexadecyltrimethylammoniumbromid verkürzt sich die Reaktionszeit auf $\leqq$ 2 Stunden, der Restgehalt an Chlorid liegt bei 0.5 %.

Selbst bei Zusätzen von < 0.5 Mol-% sind Restgehalte an organischem Chlorid erreichbar, die deutlich unter den bisher beschriebenen liegen. Phasentrennungsprobleme treten bei Anwendung des erfindungsgemäßen Verfahrens nicht auf, die das gewünschte Dihydromyrcenol enthaltende organische Phase trennt sich nach Abkühlung des Reaktionsgemisches auf Temperatur unterhalb des Siedepunktes der wäßrigen Phase, d. h. nach Abkühlung auf unter ca. 100 °C, sofort sauber ab.

Überraschend und nicht vorhersehbar war auch die Tatsache, daß bei Zusatz von 1 Mol-% eines Phasentransferkatalysators, bezogen auf Dihydromyrcenylchlorid, wie z. B. Hexadecyltrimethylammoniumbromid, nach einer Reaktionszeit von 30 Stunden keinerlei Dihydromyrcenylchlorid mehr nachweisbar ist. Des weiteren war überraschend und nicht vorhersehbar, daß die durch Phasentransferkatalysatoren beschleunigte Hydrolyse ohne Einbußen an der Selektivität ablaufen würde.

Als Phasentransferkatalysatoren kommen quarternäre Verbindungen, wie verschieden substituierte Phosphonium-, Ammonium- und Pyridiniumhalogenide, in Frage. Zu nennen wären hier u. a. beispielsweise Tetrabutylammoniumhalogenide, Tetrabutylphosphoniumhalogenide, Hexadecylpyridiniumhalogenide, Hexadecyltrimethylammoniumhalogenide. Zu den Substituenten gehören beispielsweise Tetraethyl-, Alkyltriethyl-, Phenyl-, Benzyl-, n-Octyltrimethyl bzw. -tetrabutyl usw. Als Halogenide werden insbesondere Brom und Chlor eingesetzt. Ein besonders bevorzugter Katalysator ist Hexadecyltrimethylammoniumhalogenid, insbesondere -bromid.

Hervorzuheben ist, daß die eingesetzten Phasentransferkatalysatoren wiederholt einsetzbar sind, somit ist eine erhebliche Verringerung der Abwassermenge möglich.

Das Molverhältnis (in Mol-% bezogen auf Dihydromyrcenylchlorid) an Phasentransferkatalysator kann 0.001-10 Mol-% betragen, vorzugsweise 0.05-1 Mol-%.

Der Anteil an $H_2O$ zu Dihydromyrcenylchlorid kann im Bereich von 1-100 Mol-Äquivalenten liegen.

Die Hydrolyse wird bei Temperaturen (unter Normaldruck) zwischen 10 und 100 °C durchgeführt, vorzugsweise bei 90-100 °C. Der Zusatz an basischem Puffer pro Mol Dihydromyrcenylchlorid sollte zwischen 1.0 und 5 Mol Base liegen, vorzugsweise bei 1-1.25 Mol.

Hinsichtlich der Base gibt es keine Einschränkungen, doch zeigt sich, je schwächer die Base, desto geringer der Anteil an Eliminierungsprodukten. Als Basen kommen besonders NaOH, KOH, $Ca(OH)_2$, $NaHCO_3$, $Na_2CO_3$, $Li_2CO_3$, $LiHCO_3$ und $KHCO_3$ in Frage, vorzugsweise $NaHCO_3$.

Bei dem eingesetzten Dihydromyrcenylchlorid kann es sich sowohl um reine Substanz, wie auch um ein Substanzgemisch aus verschiedenen terpenoiden Chloriden handeln. Probleme mit der Selektivität in Bezug auf Dihydromyrcenol ergeben sich hierdurch nicht.

Die mit der Erfindung zu erzielenden Vorteile bestehen insbesondere darin, daß

- eine wesentliche Verkürzung der Reaktionszeiten erreichbar ist,
- nur sehr wenig Phasentransferkatalysator benötigt wird,
- keine Phasentrennungsprobleme auftreten,
- auch Gemische aus verschiedenen terpenoiden Chloriden einsetzbar sind.

Vergleichsbeispiel

In einem 250 ml Dreihalskolben, ausgestattet mit Rührer, Thermometer und Rückflußkühler werden 27 g Dihydromyrcenylchloridgemisch (Zusammensetzung: 90 % = 24.3 g = 0.14 Mol Dehydromyrcenylchlorid; 10 % = 2.7 g = 0.02 Mol Dehydromyrcen), 8 g (0.2 Mol) NaOH und 108 ml (6 Mol) $H_2O$ vorgelegt. Die Lösung wird für 22 Stunden unter Rückflußbedingungen zum Sieden erhitzt. Danach wird die organische Phase abgetrennt, mit $H_2O$ gewaschen und getrocknet. Neben 56 % Dihydromyrcenol sind noch 4 % Dihydromyrcenylchlorid und 40 % Dihydromyrcen enthalten.
Anschließend destilliert man über eine Füllkörperkolonne das Reaktionsgemisch unter Vakuum vom Dihydromyrcen ab.

Beispiel 1

Es wurde wie im Vergleichsbeispiel gearbeitet, abweichend wurden 11.8 g (0.14 Mol) $NaHCO_3$ und 0.68 mmol (0.25 g) Hexadecyltrimethylammoniumbromid (Cetyltrimethylammoniumbromid) zugegeben. Nach 2 Stunden im Rückfluß enthielt die organische Phase 64 % Dihydromyrcenol, 0.5 % Dihydromyrcenylchlorid und 33.5 % Dihydromyrcen. Der Zusatz am Phasentransferkatalysator bewirkt also eine um über den Faktor 10 geringere Reaktionszeit bei gleichzeitiger geringer Verbesserung der Selektivität.

Beispiel 2

Es wurde wie in Beispiel 1 gearbeitet, abweichend wurden 0.068 mmol (0.025 g) Hexadecyltrimethylammoniumbromid zugegeben. Nach 7 Stunden im Rückfluß enthielt die organische Phase 64.5 % Dihydromyrcenol, 1.0 % Dihydromyrcenylchlorid und 34.5 % Dihydromyrcen. Schon der Zusatz von $< 0.05$ Mol-%, bezogen auf das Chlorid, bewirkt eine deutliche Verkürzung der Reaktionszeit.

Beispiel 3

Es wurde wie in Beispiel 1 gearbeitet, abweichend wurden 2.85 mmol (0.91 g) Hexadecyltrimethylammoniumchlorid zugegeben. Nach 30 Stunden im Rückfluß enthielt die organische Phase 62 % Dihydromyrcenol, 38 % Dihydromyrcen. Dihydromyrcenylchlorid war unter diesen Reaktionsbedingungen nicht mehr nachweisbar.

Beispiel 4

Es wurde wie in Beispiel 1 gearbeitet, abweichend wurden 0.2 mmol (0.06 g) Hexadecylpyridiniumbromid zugegeben. Nach 3 Stunden im Rückfluß enthielt die organische Phase 65 % Dihydromyrcenol, 1.5 % Dihydromyrcenylchlorid und 33.5 % Dihydromyrcen.

Beispiel 5

Es wurde wie in Beispiel 1 gearbeitet, abweichend wurden 7.3 mmol (2,8 g) Hexadecylpyridiniumbromid zugegeben. Nach 2 Stunden im Rückfluß enthielt die organische Phase 62 % Dihydromyrcenol, 0.2 % Dihydromyrcenylchlorid und 37,8 % Dihydromyrcen.

Beispiel 6

Es wurde wie in Beispiel 1 gearbeitet, abweichend wurden 13.6 mmol (4.4 g) Tetrabutylammoniumbromid zugegeben. Nach 6 Stunden im Rückfluß enthielt die organische Phase 70.0 % Dihydromyrcenol, 1 % Dihydromyrcenylchlorid und 29 % Dihydromyrcen.

Beispiel 7

Es wurde wie in Beispiel 1 gearbeitet, abweichend wurden 6.9 mmol (2.36 g) Tetrabutylphosphoniumbromid zugegeben. Nach 6 Stunden im Rückfluß enthielt die organische Phase 61 % Dihydromyrcenol, 1.5 % Dihydromyrcenylchlorid und 37.5 % Dihydromyrcen.

Beispiel 8

Es wurde wie in Beispiel 1 gearbeitet, abweichend wurden 6.2 mmol (2.8 g) Methyltrioctylammoniumbromid zugegeben. Nach 6 Stunden im Rückfluß enthielt die organische Phase 51 % Dihydromyrcenol, 0.5 % Dihydromyrcenylchlorid und 48.5 % Dihydromyrcen.

**Patentansprüche**

**1.** Verfahren zur Herstellung von Dihydromyrcenol aus Dihydromyrcenylchlorid,
dadurch gekennzeichnet,
daß bei der basischen Hydrolyse ein Phasentransferkatalysator in einer Menge von 0.001-10 Mol-%, bezogen auf Dihydromyrcenylchlorid, zugesetzt wird.

**2.** Verfahren nach Anspruch 1,
dadurch gekennzeichnet,
daß als Phasentransferkatalysatoren substituierte quarternäre Ammonium-, Phosphonium- und Pyridiniumhalogenide verwendet werden.

**3.** Verfahren nach den Ansprüchen 1 und 2,
dadurch gekennzeichnet,
daß als Phasentransferkatalysatoren Hexadecyltrimethylammoniumhalogenide eingesetzt werden.